# EUROPEAN PATENT SPECIFICATION

(11) **EP 3 160 559 B1**
(45) Date of publication and mention of the grant of the patent: **16.10.2019**
(21) Application number: 14895690.7
(22) Date of filing: 25.06.2014
(51) Int. Cl.: A61M 16/16, A61M 15/00, A61M 16/08, A61M 16/10

(54) **A MICRO-HUMIDIFIER**
MIKROBEFEUCHTER
MICRO-HUMIDIFICATEUR

(43) Date of publication of application: 03.05.2017
(73) Proprietor: Outstanding Healthcare Company Limited, Hong Kong (CN)
(72) Inventor: ZHENG, Ze Guang, Hong Kong (CN); XU, Jie Bing, Hong Kong (CN); FU, Kwok Fu, Hong Kong (CN); KOH, Ming Fai, Hong Kong (CN); TO, Ki Cheung, Hong Kong (CN); CHU, Ching Sau, Hong Kong (CN)
(74) Representative: Ward, David Ian
(86) International application number: PCT/CN2014/080687
(87) International publication number: WO 2015/196379

(56) References cited:
- WO-A1-2012/080923
- CN-A- 101 053 685
- CN-A- 103 566 444
- CN-U- 202 982 861
- US-A1- 2005 229 926
- US-A1- 2008 243 050

## Description

### Contents

1. Field of the Invention
2. Background
3. Summary of the Invention
4. Brief Description of the Figures
5. Detailed Description
   5.1 Device connection with breathing circuit and ventilator
   5.2 Different device components
   5.3 Liquid supply system in the first and the second preferred embodiment
   5.4 Micro-humidifier operation
   5.5 Temperature and Humidity Feedback system

### 1. Field of the Invention

A humidifier device may humidify a gas, more specifically humidify a gas delivered to a patient under mechanical ventilation.

### 2. Background

Humidifiers are often used in conjunction with, for example, mechanical ventilation systems to assist patients who require humidified air in order to assist their breathing. This may be in a hospital, or at a patient's home.

With mechanical ventilation systems, to prevent the overstimulation and/or damage to the patient's air tract, which may lead to excessive secretions that block the airway, it is sometimes preferred that the gases delivered to patient should be about 37 °C and have a relative humidity of up to 100%. It is also believed that water droplet of appropriate size will have greater chance to deposit in the bronchiole and alveoli

It is further believed that the lung deposition characteristics and efficacy of an aerosol depend largely on the particle or droplet size. Generally, the smaller the particle, the greater its chance of peripheral penetration and retention within the lungs. However, for very fine particles below 0.5 µm in diameter there is a chance of avoiding deposition altogether and being exhaled. In 1966 the Task Group on Lung Dynamics, concerned mainly with the hazards of inhalation of environmental toxins, proposed a model for deposition of particles in the lung. This suggested that particles of more than 10 µm in diameter are most likely to deposit in the mouth and throat, for those of 5-10 µm diameter a transition from mouth to airway deposition occurs, and particles smaller than 5 µm in diameter deposit more frequently in the lower airways and are appropriate for pharmaceutical aerosols.

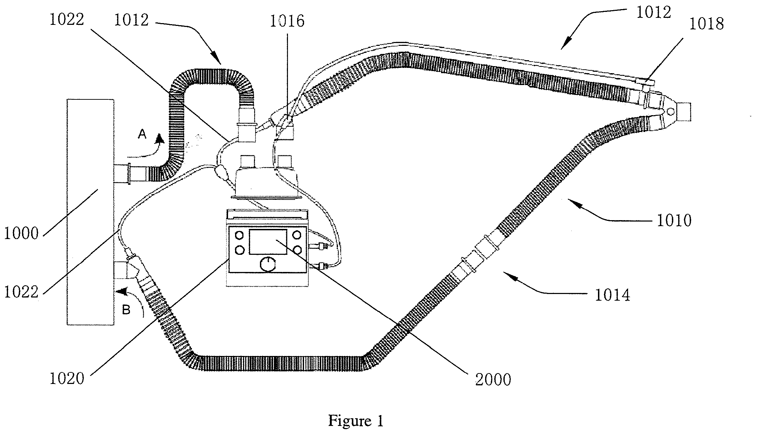

Currently, typical respiratory humidifiers (e.g., Model MR850 from Fisher Paykel Healthcare) and heated breathing circuits (see, e.g., Figure 1) are used with ventilators to heat and humidify the gases delivered to the patient.

However, it has been found that in the typical humidifier/mechanical ventilator systems the tidal volume setting is variable while the humidification rate of a respiratory humidifier is fixed. Therefore, when the tidal volume increases, the relative humidity typically cannot reach 100% relative humidity because of the air surge. In contrast, when the tidal volume decreases, water condensation occurs in the breathing circuits due to the lowered air pressure.

When relative humidity is less than 100%, the gas condition is not favourable to humidify the patient's airway and lungs; the presence of condensation inside the breathing circuit is a risk factor for ventilator-associated pneumonia. Both can lead to the occurrence of pneumonia, the failure of the therapy, delay the ventilator weaning process, increase the mortality rate, growth of microbes and mold in condensed water, increased therapy complications and/or cost.

In addition, current humidifying devices, for example, those used in a breathing circuit, employ significant amounts of energy to heat up and evaporate large amounts of water in their humidification chambers. These devices are also quite heavy and bulky and therefore need to be placed far away from the patient so as not to unduly weigh upon the breathing circuit. Such a location significantly increases the amount of condensation formed as well as the loss of temperature as the humid, heated air travels down the breathing circuit towards the patient. This often means that additional heating elements need to be placed in the tube so as to keep the air warm and the water suspended and to reduce condensation. Such a set up also increases the complexity of the system and the amount of energy needed to provide acceptably heated, humid air for the patient to breathe.

US 2005/229926 discloses a nebulizer for operably connecting to a respiratory circuit, the nebulizer comprising a medicament port, a reservoir and an aerosol generator.

WO2012/080923 describes a humidification unit including a liquid chamber, a nebulizer that nebulizes liquid from the liquid chamber, an aerosol chamber, and a heat source (309a) that converts aerosolized liquid into a vapor that humidifies gas in a patient circuit.

Accordingly, there is a need for an improved system and humidifier to solve the above problems. It would be desirable to be able to better control the quality (e.g., vapour particle size, temperature, humidity, etc.) of humid air, preferably humid and heated air, administered to a patient via a breathing circuit. It would be desirable to provide a humidifier and/or a system which would provide a more constant humidity during tidal volume increases while avoiding condensation when tidal volume decreases.

### 3. Summary of the Invention

According to a first aspect of the invention there is provided a micro-humidifier in accordance with claim 1.

According to a second aspect of the invention there is provided a respiratory humidification system according to claim 12.

Some embodiments of the invention are described by the dependent claims.

According to the present invention, a micro-humidifier for operably-connecting to a breathing circuit contains a liquid feed, a liquid chamber operably-connected to the liquid feed, and a vapour generator operably-connected to the liquid chamber. The liquid chamber has a volume of from about 0.01 mL to about 30 mL. The micro-humidifier is operably-connected to and/or designed to be operably-connected to the breathing circuit upstream of the patient. When the micro-humidifier is operably-connected to a breathing circuit, the vapour from the vapour generator enters into the breathing circuit.

According to the present invention a respiratory humidification system contains a breathing circuit for operably-connecting a patient and a micro-humidifier. The micro-humidifier as described herein. The micro-humidifier is operably-connected to the breathing circuit upstream of the patient, and the vapour from the vapour generator enters into the breathing circuit.

Without intending to be limited by theory, it is believed that the new micro-humidifier device developed by the inventors may address one or more of the problems discussed above. Especially, the invention may address existing problems when used in invasive and non-invasive mechanical ventilation. It is believed that the micro-humidifier device and/or the breathing system described herein is capable of supplying gases with various temperature, humidity and/or water droplet sizes. it is believed that the device can reduce or even prevent the occurrence of condensation, reduce microbe and mold growth, and/or lower the cost of respiratory humidification.

More specifically, and without intending to be limited by theory, it is believed that such a respiratory humidification system and/or micro-humidifier provides surprising benefits such as a reduction or even the prevention of condensation, reduced risk of ventilator-associated pneumonia, reduced energy requirements, reduced system complexity, reduced chance of microbial, and/or mold growth in the respiratory circuit, overall reduced therapy complications and cost, better control over the air humidity and/or temperature actually being inhaled by the patient, etc.

It is believed that such a small and light micro-humidifier enables the humidification source to be located very close to where the patient breathes in the air from the breathing circuit. This reduces or even eliminates the need for additional heating elements along the inspiratory limb of the breathing circuit, as there is little or no chance for water vapour to condense prior to being inhaled by the patient. This in turn reduces energy required, and also the chances of microbial and/or mold growth. In addition, as the micro-humidifier itself is so close to the patient, it is believed that the micro-humidifier can react more quickly to tidal volume fluctuations and thereby provide improved control of the humidity and/or the temperature of the air inhaled by the patient. Such a system may also allow better control of the quality of the vapour being inhaled by the patient.

### 4. Brief Description of the Figures

Figure 1 is a schematic diagram of the typical representative, existing humidifier and breathing circuit system.
Figure 2 is a schematic diagram of the breathing circuit and micro-humidifier of the present invention.
Figure 3 is a partially-cut-away, schematic diagram of an embodiment of the present invention.
Figure 4 is a partially-cut-away, schematic diagram of an alternate embodiment of the present invention.

The figures herein are for illustrative purposes only and are not necessarily drawn to scale.

### 5. Detailed Description

Unless otherwise specifically provided, all tests herein are conducted at standard conditions which include a room and testing temperature of 25 °C, sea level (1 atm.) pressure, and pH 7, and all measurements are made in metric units. Furthermore, all percentages, ratios, etc. herein are by weight, unless specifically indicated otherwise.

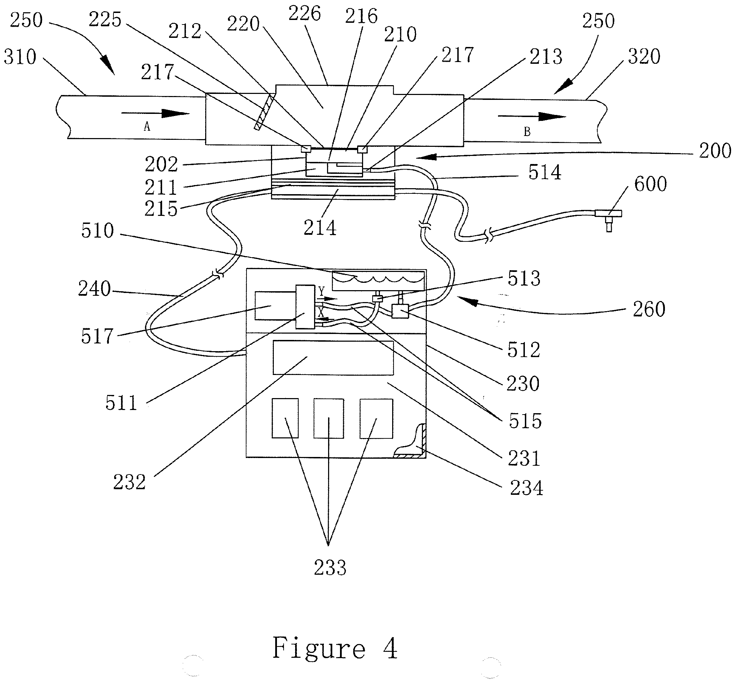

As used herein, the term "upstream" indicates the direction that the flow (e.g., air flow, liquid flow, etc.) is coming from. Conversely, as used herein, the term "downstream" indicates the direction that the flow is going. In the figures herein, the air flow is indicated with arrows A, B and sometimes C. In these figures, A is always upstream of B which in turn is upstream of C (when present). Conversely, C (when present) is always downstream of B which is in turn downstream of A. In Figure 4, X is upstream of Y when indicating the direction of the liquid flow.

As used herein, the term "operably-connected" means that the indicated items are connected in a way that, based on a full reading of this specification, one skilled in the art would normally connect them so that the items work together in the manner indicated and/or intended.

Breathing circuit and humidifiers are known as shown in Fig. 1 where a ventilator (1000) is operably-connected to a breathing circuit (1010). One skilled in the art understands that the breathing circuit (1010) is for providing and conducting the air along a predefined path, whereas the ventilator (1000) is for assisting breathing by pushing the air through the breathing circuit (1010). So while the breathing circuit (1010) itself typically does not include a ventilator (1000), it is usually attached to a ventilator (1000). The breathing circuit (1010) contains an inspiratory limb (1012), and an expiratory limb (1014). A humidifier, (2000) is operably-connected to the breathing circuit upstream of the patient and provides humidified, and often heated air to the patient. A first inspiratory sensor (1016) is located near the humidifier, while a second inspiratory sensor (1018) is located near the patient. Both the first inspiratory sensor and the second inspiratory sensor are operably-connected to the control device (1020). The inspiratory sensors typically detect both the temperature and the humidity of the gas flowing by them and this data is collected and analyzed by the control device (1020). The control device (1020) also provides heating element control via heating control wires (1022). The expiratory limb (1014) is downstream of the patient and provides an air path leading away from the patient. Both the inspiratory limb (1012) and the expiratory limb (1014) often contain heating elements (not shown) therein to reduce condensation in the breathing circuit. In Figure 1, arrows A and B show the direction of gas flowing through the breathing circuit.

### 5.1 General description of the device connection with breathing circuit and ventilator

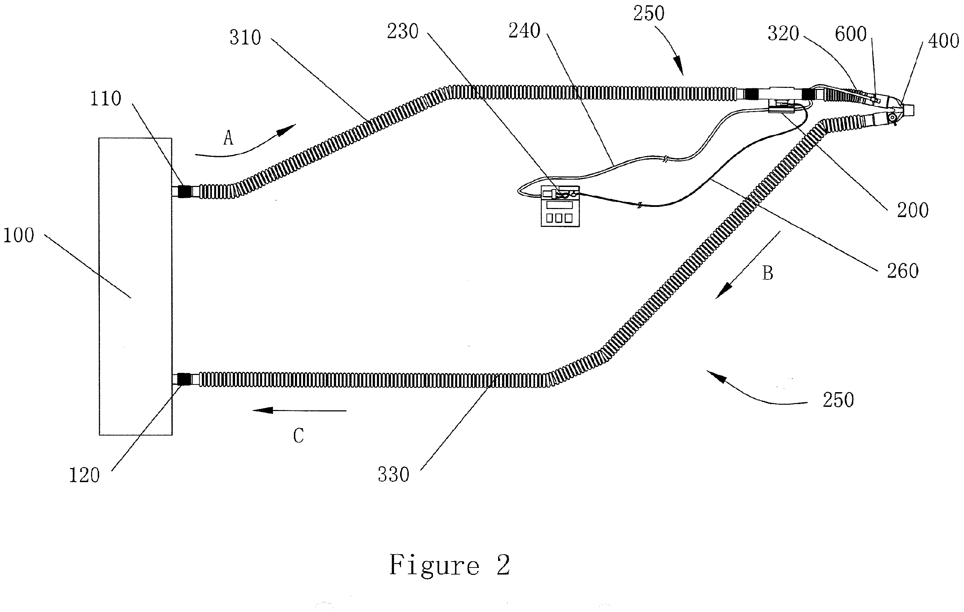

As shown by an embodiment of the invention in Figure 2, the micro-humidifier (200) is installed in the breathing circuit (250) operably-connected with the ventilator (100).

The ventilator has an inspiratory port (110) and an expiratory port (120). The inspiratory port (110) is used to supply cold gas (usually dry oxygen or ambient air) to assist patient breathing. The breathing gas comes out of the inspiratory port (110) and passes through the inspiratory limb (310, 320) to the patient; The exhaled gas from the patient passes through expiratory limb (330) and expiratory port (120) and back to the ventilator (100). The arrows A, B, and C show the direction of the gas passing through the breathing circuit (250).

In Figure 2, the micro-humidifier (200) is designed to be operably-connected to the inspiratory limb (310, 320) of a breathing circuit such as shown. The inspiratory limb typically consists of two pieces of connection tubing. The first connection tubing (310) is connected between the ventilator and the micro-humidifier, and the second connection tubing (320) is connected between the micro-humidifier and the patient end Y-connector (400). Such an arrangement is possible with the present invention because the micro-humidifier (200) is so small and/or light as to be relatively low weight in comparison to the rest of the breathing circuit (250), and/or to be so small and/or of such a low weight as to not hinder the patient.

In Figure 2, the second connection tubing (320) is shorter than the first connection tubing, so that the micro-humidifier (200) is closer to the patient as compared to the system in Figure 1. This allows the heated and humidified gas mixture to be inhaled by patient soon after mixing. This in turn reduces and/or avoids the potential for condensation to occur in the second connection tubing (320), thus reducing and/or preventing bacteria and mold growth that could affect the therapy. Without intending to be limited by theory, it is believed that such a position close to the patient further allows the micro-humidifier, especially in conjunction with a sensor (600) and a control device (230), to more quickly detect and react to fluctuations in the humidity brought on by tidal volume fluctuations as the patient breathes. The micro-humidifier may therefore immediately either increase or decrease the amount of water vapour supplied to the breathing circuit immediately prior to (in both time and distance) the inhalation of humidified gas by the patient. This in turn evens out the humidity fluctuations which would occur during tidal volume fluctuations, so as to further reduce both condensation and low humidity conditions during use. Without intending to be limited by theory, we believe that as such a micro-humidifier is heretobefore unknown, that such a location and benefit would not have been possible nor conceivable with the previous large, heavy humidifiers used in the art.

In the embodiment of Figure 2, the micro-humidifier (200) and the first connection tubing (310) are detachable. Furthermore, in an embodiment herein except for the control device (230) and the sensor (600), the other parts of the micro-humidifier and/or breathing circuit are disposable so as to meet the hygiene requirements in a hospital. It is worth noting that in many cases the first connection tubing is used to delivery relatively dry oxygen or other gas, so the risk of condensation occurrence and bacteria growth is minimal. In this case, the first connection tubing may require less frequent changing, thus reducing the cost of therapy. In Figure 2, the sensor (600) may be, for example, a temperature sensor, a humidity sensor, an air flow rate sensor, or a combination thereof. In a preferred embodiment herein the sensor (600) contains both a temperature sensor and a humidity sensor. In an embodiment herein, the sensor is placed immediately before the patient, for example, immediately prior to the Y-connector (400) so as to more accurately measure the properties of the gas which is inhaled by the patient. In an embodiment herein, there is no first inspiratory sensor (see Figure 1 at 1016) located near the micro-humidifier, as it is not needed. Thus, in Figure 2, there is only a single sensor (600) corresponding to the second inspiratory sensor (1018) in Figure 1. This further reduces cost and complexity, as the operator does not need to worry about inserting the wrong sensor at the wrong point.

In Figure 2, a control wire (240) connects the control device (230) with the micro-humidifier (200). The control wire (240) typically passes current to the micro-humidifier (200) to power it, and/or transmits data and instructions to the micro-humidifier and/or the various sensors. In addition, a liquid feed (260) connects the control device (230) and the micro-humidifier (200). This liquid feed (260) provides a liquid, typically water, to the micro-humidifier to be vaporized. Without intending to be limited by theory, it is believed that such an arrangement of the control device and the liquid feed further reduces the size and/or weight of the micro-humidifier and allows it to be attached, and even suspended, close to the patient without being either too heavy or cumbersome.

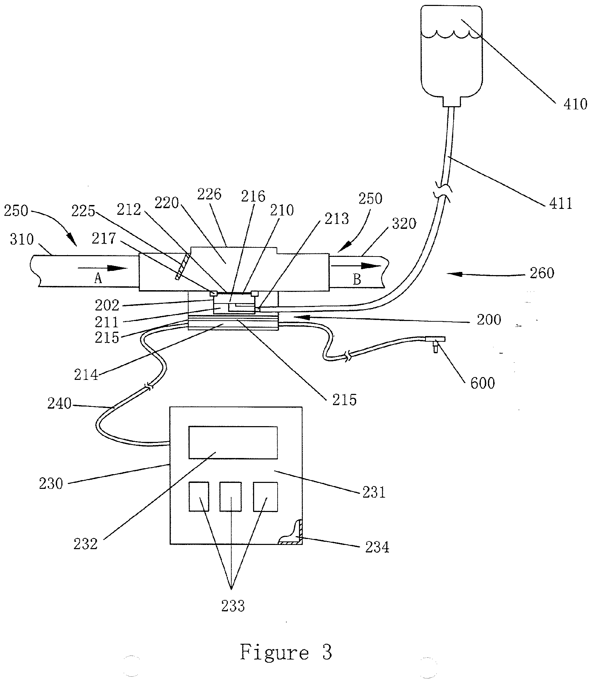

Figures 3 and 4 show various non-limiting, preferred embodiments of the micro-humidifier structure.

### 5.2 Detailed Description of different device components

In Figures 3 and 4, the micro-humidifier (200) contains a vapour generator (202) which in this embodiment contains a nebulizer which in turn contains a nebulizer plate (212). The micro-humidifier contains a liquid chamber (216) covered by a nebulizer plate (212). When the nebulizer plate (212) vibrates, the liquid in the liquid chamber (216) is nebulized to vapour (not shown). In Figure 2, the liquid is also heated by the heater (211) prior to being nebulized. Arrows A and B indicate the direction of the gas flowing through the breathing circuit (250).

The nebulizer plate (212) releases the vapour into a mixing channel (220), positioned between the first connection tubing (310) and the second connection tubing (320). The mixing channel (220) provides a space for the gas, typically colder, drier gas, from the ventilator (see Figure 2 at 100) to mix with the vapour, preferably heated vapour, generated by the nebulizer plate (212). The mixing channel (220) contains a baffle (225) and venting arrangement (226) which induces turbulent airflow so as to better mix the vapour with the gas from the ventilator. Such a baffle (225) can be any structure which is added so as to increase the turbulence of the airflow and thereby improve the mixing of the vapour and the gas from the ventilator. The venting arrangement (226) includes an increased headspace which allows the vapour to more easily mix with the gas so as to avoid overly-high local humidity concentrations which could lead to sudden condensation.

The micro-humidifier also contains a control device (230), which controls, for example, the level of heating and nebulization such that an adequate humidification is provided to patient. Specifically, the control wire, (240) operably-connects the control device (230) with the micro-humidifier (200). The control wire (240) typically passes current to the micro-humidifier (200) to power it, and/or transmits data and instructions to the micro-humidifier and/or the various sensors. In addition, in Figure 4, a liquid feed (260) is operably-connected to the control device (230) and the micro-humidifier (200). This liquid feed (260) provides a liquid, typically water, to the micro-humidifier to be vaporized.

In the micro-humidifier (200) includes the liquid chamber (216) and the mixing channel (220) which are integrated and interconnected to the micro-humidifier; also, one end of the mixing channel (220) is connected to the first connection tubing (310), and the other end of the mixing channel (220) is connected to the second connection tubing (320).

In the micro-humidifier (200), the liquid chamber (216) is operably-connected with a liquid feed (260) via a liquid connection port (213) which is where the liquid first enters the liquid chamber (216). The liquid chamber (216) is typically a water-tight but not vapour-tight chamber which bounded by the upstream liquid connection port (213) and the downstream vapour generator (202). The liquid feed (260) provides a small, but preferably constant, amount of liquid, typically water either with or without a drug, to the liquid chamber (216) where the liquid is temporarily stored prior to nebulization. In Figure 3 the bottom of the liquid chamber (216) contains a heating element (211) which heats up the liquid prior to nebulization. However, the inventors recognize that it is possible that the heating element (211) is located in a different portion of the liquid chamber (216) and/or micro-humidifier (200), while still providing similar and/or comparable results.

In Figures 3 and 4, the nebulizer plate (212) is located between the liquid chamber (216) and the mixing channel (220), when the nebulizer plate (212) vibrates, the liquid in the liquid chamber (216) will be nebulized and enter the mixing channel (220) for delivery to the patient.

In Figures 3 and 4, a circuit board (214) is located at the bottom of the micro-humidifier (200) to control the heating element (211) and nebulizer plate (212). Thus, the circuit board is operably-connected to the heating element (211) and the nebulizer plate (212). However, it is recognized that the circuit board may not be in the micro-humidifier (200) itself, but may instead be located in, for example, the control device (230), or both.

In Figures 3 and 4, a heat insulation plate (215) is installed between the heating element (211) and the circuit board (214) to prevent the heating element (211) from harming the circuit board (214) after prolonged use.

In Figures 3 and 4, a gasket (217) ensures a good seal between the vapour generator (202) and the mixing channel (220) and reduces the chances of vapour leaking from the breathing circuit (250). This helps to ensure that the humidified gas is properly delivered to the patient and also reduces the risk of the micro-humidifier (200) becoming accidentally disconnected from the mixing channel (220).

In Figures 3 and 4, the control device (230) contains a main body (231). The main body consists of a circuit board (234), operably-connected to a display (232) and buttons (233) for controlling the micro-humidifier. The display (232) may provide information such as temperature, humidity, vaporisation rate, air flow rate, alerts, etc. to the patient and/or a health care professional such as a nurse or doctor.

In Figure 3, the micro-humidifier also contains liquid feed (260) which further contains an external liquid supply (410), operably-connected via tubing (411) to the liquid connection port (213) on the side of the micro-humidifier (200). The liquid connection port (213) is close to and operably-connected to the liquid chamber (216).

### 5.3 Liquid supply system in the first and second preferred embodiment

The major difference between the embodiments in Figure 3 and Figure 4 is the mechanism used to supply liquid to the nebulizer.

Figure 3 shows an embodiment where the liquid for nebulization comes from an external liquid supply (410), which may be an infusion set up common in hospitals, such as commonly-referred to as an IV-bag, an infusion set-up, an IV-bottle, etc. Thus, the actual external liquid supply container may be a bag, a bottle, etc. The liquid feed (260) further contains tubing (411) operably-connected to the liquid connection port (213). The external liquid supply (410) is typically gravity-fed and is hung in a position higher than the micro-humidifier (200) so that the liquid goes down the tubing (411) to the micro-humidifier (200). Without intending to be limited by theory it is believed that an advantage of this embodiment is the simplified liquid supply system, as well as a reduced need for electricity.

In contrast, Figure 4 shows another embodiment where the liquid for nebulization comes from a liquid feed (260) which is embedded in the control device (230). The liquid feed (260) has a first connection tube (515) which operably-connects, in order, the internal tank (510), a peristaltic pump (511) and a relief valve (512). A second connection tube (514) operably-connects the relief valve (512) with the liquid connection port (213). While a peristaltic pump is preferred in this embodiment, the inventors recognize that other types of pumps are useful herein as well. However, due to the low energy requirements, the low pressure needed, and the low noise, a peristaltic pump is a preferred pump herein. Arrows X and Y indicate the direction of the liquid flow through the liquid feed (260).

The internal tank (510) contains liquid for nebulization. In an embodiment herein a filter (513) is provided between the internal tank (510) and the peristaltic pump (511). In an embodiment herein, the exit of the relief valve (512) is operably-connected back to the internal tank (510).

Typically, the peristaltic pump is driven by a motor (517), which is typically operably-connected to and controlled by the circuit board (234). In such an arrangement, liquid is constantly pumped out of the internal tank (510), then passed through the relief valve and the second connection tube (514) to the liquid chamber (216). When the liquid chamber (216) is full, the liquid output of the peristaltic pump (511) flows back to the tank via the relief valve (512).

Without intending to be limited by theory it is believed that the embodiment of Figure 4 is more suitable for use in, for example, an Intensive Care Unit, because despite its relatively complex structure, the device's operation is easy.

### 5.4 Detailed description of the micro-humidifier operation

Typically a ventilator only outputs a relatively dry and cold gas that passes through the first connection tubing to the mixing channel where the micro-humidifier is operably-connected. The micro-humidifier releases vapour, typically water vapour, and preferably heated water vapour into the mixing channel; or directly into the mixing channel; where the humidified gas; or humidified and heated gas; is then carried through the second connection tubing to the patient. In an embodiment herein the breathing circuit contains a first connection tubing operably-connected upstream of the mixing channel and a second connection tubing operably-connected between the mixing channel and the patient. Without intending to be limited by theory it is believed that such a second connection tubing; or a short second connection tubing; allows for stabilization of the mixed gasses prior to their inhalation by the patient.

### 5.5 Temperature and Humidity Feedback System

The control device may contain a circuit board, preferably a printed circuit board, and humidity control circuitry operably-connected with the circuit board and the nebulizer plate. The control device may also contain a circuit board; or a printed circuit board, containing temperature control circuitry operably-connected with the circuit board and a heating element.

The control device may contain a circuit board, preferably a printed circuit board, and motor control circuitry operably-connected with the motor of the peristaltic pump.

If the control device and the nebulizer in the micro-humidifier are separated, they may communicate with each other in any way, such as, for example, via a control line (240) which transmits data and instructions therethrough, or even wirelessly The control device may control, for example, one or more variables affecting the quality of the gas (for example, one or more variable such as the temperature, humidity, vapour droplet size, air flow rate, etc.) provided to a patient. In an embodiment herein the control device monitors and regulates the temperature of the gas provided to the patient, and/or the humidity of the gas provided to the patient; or the control device monitors and regulates the temperature of the gas provided to the patient; or the control device monitors and regulates the humidity of the gas provided to the patient.

The micro-humidifier and/or the control device may further include a sensor (600); or a temperature sensor, a humidity sensor and/or an air flow rate sensor; or a humidity sensor; or a temperature sensor and a humidity sensor; or a temperature sensor, a humidity sensor and an air flow rate sensor, operably-connected with a circuit board.

In an embodiment herein, the micro-humidifier (200) contains a circuit board (214) and the control device (230) also contains a separate circuit board (234). Theses circuit boards are typically in operably-connected via the control line (240). In such a case as seen in Figures 3 and 4, the sensor (600) may first be operably-connected first to the circuit board (214) in the micro-humidifier (200) and then to the circuit board (234) in the control device (230).

The sensor typically monitors both the temperature and humidity of gas provided to the patient end of the breathing circuit, either at the Y-connector (see Figure 2 at 400) or immediately prior to the Y-connector (see Figure 2 at 400), and then feeds back data and sensor readings to the circuit board (214 or 234). The readings may then be shown on the display (232). The operator may adjust the control device's (230) settings via the buttons (233), and/or the control may be automatic. In an embodiment herein, the control device (230) contains one or more buttons (233) to control the control device (230). In an alternate embodiment herein the control device (230) contains a touch screen interface which combines the display (232) with a software-defined control panel. In an alternate embodiment herein, the control device (230) contains a dial-type control.

In an embodiment herein, when the sensor (600) detects that the gas humidity at the patient end is below target, then the control device (230) may instruct the nebulizer plate (210) to increase the level of nebulization. Alternatively, if the sensor (600) detects that the humidity is so high as to be likely causing condensation, then the control device (230) may instruct the nebulizer plate (210) to reduce the level of nebulization.

In an embodiment herein when the sensor (600) detects that the gas temperature at the patient end is low, the control device (230) may instruct the heating element (211) to increase the level of heating. Alternatively, if the sensor (600) detects that the gas temperature at the patient end is high, the control device (230) may instruct the heating element (211) to reduce the level of heating.

As noted above, the micro-humidifier is typically installed in the inspiratory limb of a breathing circuit. The inspiratory limb consists of the first connection tubing used to operably-connect the ventilator to the micro-humidifier and the second connection tubing used to operably-connect the micro-humidifier to the patient Y-connector.

Further preferred, non-limiting features are described herein, and especially below. It is specifically noted that these features may be applied individually or combined in any fashion in the present invention by one skilled in the art and yet still remain within the scope of the present invention.

In an embodiment herein, the micro-humidifier further contains a mixing channel downstream of the vapour generator. In an embodiment herein, the mixing channel may be contained within a T-connector. The mixing channel will typically be upstream of the patient. In an embodiment herein the micro-humidifier is attached to the breathing circuit at the mixing channel, preferably via a gasket. Without intending to be limited by theory, it is believed that the mixing channel provides space for the heated vapour generated to quickly and effectively mix with cold gases from the ventilator so as to reduce micro-concentrations of supersaturated air which could lead to condensation within the breathing circuit.

In an embodiment herein the mixing channel contains a baffle, a venting arrangement, or a combination thereof.

In an embodiment herein, the micro-humidifier is detachable from the inspiratory limb.

In an embodiment herein, a gasket is installed between the nebulizer plate and the mixing channel. Without intending to be limited by theory, this is especially preferred if the mixing channel is detachable from the rest of the micro-humidifier and/or the vapour generator.

In an embodiment herein, the micro-humidifier has a nebulizer plate which can nebulize liquid by vibration.

Without intending to be limited by theory, we believe that there is trade-off when designing the liquid chamber. The volume of the liquid chamber should be large enough to ensure sufficient supply of liquid for nebulization; however, the inventors recognize that if the volume is too large, then the micro-humidifier will be too heavy and drag on the breathing circuit. Therefore in an embodiment herein, the liquid chamber has a volume of from about 0.01 mL to about 30 mL; or from about 0.05 mL to about 20 mL; or from about 0.1 mL to about 15 mL; or from about 0.1 mL to about 10 mL; or from about 1 mL to about 7 mL; or from about 2 mL to about 5 mL.

In an embodiment herein the "micro-humidifier weight" (without the control device and liquid feed as described below) is less than or equal to about 300 g; or from about 300 g to about 1 g; or from about 100 g to about 5 g; or from about 75 g to about 10 g. For the sake of clarity, the micro-humidifier weight as defined herein does not include the weight of the control device, sensors, wires operably-connecting the sensors, or any control wire(s). As used herein, the micro-humidifier weight includes the estimated full weight of any liquid from the liquid connection port to the vapour generator and therebetween including the weight of liquid in the liquid chamber. The micro-humidifier weight also includes the weight of the mixing channel, but does not include the weight of the breathing circuit such as the inspiratory limb(s), the Y-connector, and the expiratory limb(s). The weight of liquid herein is assumed to be 1g/ml. However, the weight of the liquid feed external to the liquid connection port, such as the tubing and the external liquid supply and the liquid therein is not included in the micro-humidifier weight described in this paragraph. If the micro-humidifier contains a circuit board therein, then this weight is also included in the micro-humidifier weight. The micro-humidifier weight does not include the weight of any mixing channel, or gasket. The intent for this definition is to define the weight of the micro-humidifier as that weight which would be solely suspended from the breathing circuit at the mixing channel.

Without intending to be limited by theory, the inventors believe that having such a low micro-humidifier weight is beneficial in that it achieves adequate functionality and yet allows the attachment of the micro-humidifier to the breathing circuit without causing significant additional strain to the patient. For example, if the weight is too high, then the micro-humidifier may drag down the breathing circuit near the patient; often patients using breathing circuits may be frail and in poor physical condition. This in turn may cause additional problems such as neck strain, breathing impairment, etc. While as light a weight as possible may be desirable, certain practical realities require that the micro-humidifier have some weight to actually work as intended.

In an embodiment herein, the micro-humidifier is operably-connected to the breathing circuit at a distance of less than or equal to about 100 cm; or from about 100 cm to about to about 1 cm; or from about 50 cm to about 2 cm; or from about 40 cm to about 4 cm from the patient. As described herein, the "distance from the patient" is measured according to the path in the breathing circuit between the portion of the vapour generator closest to the patient to the location in the breathing circuit where the patient inhales the gas. This will often be the length of the second inspiratory limb plus that of the Y-connector. In some cases this may also include the length of a mask, or a mouthpiece, or the equivalent. One skilled in the art understands that this is typically measured as the length of tubing between the vapour generator and the patient. In an embodiment herein, the above distance is provided, and a control device is also provided, where the control device includes a sensor, such as a temperature sensor, a humidity sensor, an air flow sensor, and/or a combination thereof. Without intending to be limited by theory, it is believed that such a close distance, especially in combination with the control device above, allows the micro-humidifier to be able to quickly react to increase and/or decrease the humidity and/or temperature in response to the tidal volume increases and decreases, so as to keep the humidity more constant and/or to reduce condensation in the breathing circuit.

In an embodiment herein, the respiratory humidification system contains a control device. In an embodiment herein the control device contains a feature selected from the group of a pump system, a temperature sensor, a humidity sensor, an air flow sensor, a timer, an alarm, a circuit board, a display screen, a control panel, a power source, and a combination thereof; or a pump system, a temperature sensor, a humidity sensor, a timer, an alarm, a circuit board, a display screen a control panel and a combination thereof; or a pump system, a temperature sensor, a humidity sensor, a circuit board, a display screen and a combination thereof. The various sensors may be operably-connected in any way, such as directly or indirectly to the control device, so long as the necessary data from the sensors is in some way received by the control device, or a circuit board in the micro-humidifier and/or the control device.

In an embodiment herein, the control device contains both a temperature sensor and a humidity sensor. The micro-humidifier contains a heating element in the liquid chamber and a nebulizer plate. The circuit board is operably-connected to the temperature sensor, the humidity sensor, the nebulizer plate and the heating element. In the embodiment, the circuit board receives temperature data from the temperature sensor and employs the temperature data to regulate the heating element. The circuit board further receives humidity data from the humidity sensor and employs the humidity data to regulate the nebulizer. Without intending to be limited by theory, it is believed that such a system provides improved gas quality to the patient while minimizing operational complexity for the care giver and/or patient.

In an embodiment herein, the micro-humidifier and/or the control device comprises a power source. In an embodiment herein the power source is the control device. In an embodiment herein the power source is a battery and/or a fuel cell. In an embodiment herein the power source is a plug operably-connected to a power grid, such as an AC power grid. In an embodiment herein the power source includes a plug for a normal power grid as well as a DC battery which continues to supply power in an emergency when the power grid is not working.

Any type of vapour generator may be useful herein so long as it is small, lightweight and/or energy-efficient. In an embodiment herein the vapour generator is selected from a nebulizer, a heating element, a sonication apparatus, an electrospray, and a combination thereof; or a nebulizer, a heating element, and a combination thereof; or a nebulizer. In an embodiment herein the nebulizer is selected from an oxygen nebulizer, an ultrasonic nebulizer, a compressed air nebulizer, and a combination thereof; or an ultrasonic nebulizer which converts liquids into a fine spray of aerosols. Typically, an ultrasonic nebulizer will contain a nebulizer plate which vibrates at a high frequency (for example, nebulizer plates vibrating at a frequency of from about 100 kHz to about 140 kHz are known) to cause water to become small suspended water particles and/or gaseous vapour. In an embodiment herein the vapour generator is a heating element, which typically heats water to the boiling point where it then becomes water vapour. This may later condense into fine water droplets, typically suspended water droplets, in the breathing circuit and prior to being inhaled by the patient/user. Without intending to be limited by theory, it is believe that the above types of vapour generators are especially effective given their weight-to-vaporization ratio and are energy-efficient as well.

In an embodiment herein, the vapour generator produces at least or equal to about 33 mg water vapour per liter of air at 37 °C; or from about 33 mg water vapour per liter of air to about the water vapour saturation point per liter of air at 37 °C.

In the present invention, the micro-humidifier (200) may be either permanently-connected to the mixing channel (220), or may be detachably-connected to the mixing channel (220) as preferred. In an embodiment herein the micro-humidifier is detachable from the breathing circuit. In an embodiment herein, the chamber of the micro-humidifier is integrated with and permanently interconnected with the mixing channel. One end of the mixing channel is operably-connected to the first connection tubing, and the other end of the mixing channel is operably-connected to the second connection tubing. In another embodiment herein, the micro-humidifier and the mixing channel are designed to fit together via, for example, a screw-type closure, a snap-fit closure, a lock and key-type closure, etc. In an embodiment herein the closure between the micro-humidifier and the mixing channel is substantially air tight.

In an embodiment herein, the vapour generator is a nebulizer and the micro-humidifier further contains a heating element, which may be separate from the vapour generator. The heating element may heat either the liquid, the gas, or both. In an embodiment herein the heating element heats the gas after the vapour has been added to/suspended in the gas. In another embodiment herein the heating element is located in the liquid chamber and heats the liquid prior to it being vaporized by the vapour generator. In an embodiment herein, the heating element is located in the breathing circuit; or in the breathing circuit downstream of the micro-humidifier. Without intending to be limited by theory, it is believed that by using a combination of a heating element and a nebulizer plate, the production cost and manufacturing complexity of the micro-humidifier can be lowered and while at the same time providing acceptable heating and nebulization performance and/or control.

In an embodiment herein the liquid chamber of the micro-humidifier is used for storage, typically temporary storage, of liquid obtained from the external liquid supply. In an embodiment herein, the nebulizer plate is positioned in the liquid chamber opposite a heating element.

In an embodiment herein, the bottom of the micro-humidifier contains a circuit board to control and/or drive the heating element and nebulizer plate

In an embodiment herein, the micro-humidifier contains a circuit board and a heating element. The micro-humidifier further contains a heat insulation plate between the heating element and the circuit board.

In an embodiment herein, the micro-humidifier contains a control device, which has a main body consists of a circuit board, a display and a control panel, preferably the control panel containing one or more buttons. The display and the control panel, and preferably the buttons as well, are operably-connected to the circuit board. In an embodiment herein the control panel contains a touch screen interface which is operably-connected to the circuit board.

In an embodiment herein, the liquid feed is at least partially embedded inside the control device. In an embodiment herein the liquid feed contains, in order, a first connection tube operably-connecting an internal tank, a peristaltic pump and a relief valve. In this embodiment, a second connection tube operably-connects the relief valve to the liquid connection port of the liquid chamber.

In an embodiment herein, a filter is present in the liquid feed.

In an embodiment herein, the liquid inside an internal tank is pumped to the liquid chamber by a peristaltic pump, and a filter is installed between the tank and the peristaltic pump.

In an embodiment herein, the circuit board contains temperature control circuitry operably-connected to, or in, a circuit board and a heating element to regulate the gas and/or liquid temperature

In an embodiment herein, the circuit board contains humidity control circuitry operably-connected to a nebulizer plate to regulate the vapour generation and/or the gas humidity.

In an embodiment herein, the liquid feed contains a peristaltic pump and the control device contains a circuit board. Furthermore, the circuit board is operably-connected to and controls the peristaltic pump.

In an embodiment herein, the micro-humidifier and/or the control device contains a temperature sensor and a humidity sensor to detect the gas quality at the patient end of the breathing circuit. The temperature and humidity sensor may be combined into a single sensor as desired and this single sensor may include further sensors as well. The temperature sensor and a humidity sensor are operably-connected to a circuit board in the control device and/or the micro-humidifier.

In an embodiment herein the micro-humidifier and/or the control device contains multiple temperature sensors, and/or multiple humidity sensors.

In an embodiment herein, the connection between the nebulizer plate and mixing channel further comprises a gasket. Without intending to be limited by theory, it is believed that the gasket helps to avoid the direct contact of the two parts, improve the seal therebetween, and/or to prevent the leakage of the vapour, preferably heated vapour.

In an embodiment herein, the heating element is a heater wire, a copper tube and/or a heater block.

In an embodiment herein the nebulizer plate is an ultrasonic microbore nebulization plate, preferably having a microbore diameter of from about 0.01 microns to about 5 microns; or from about 0.05 microns to about 4 microns; or from about 0.1 microns to 3 microns. Without intending to be limited by theory, it is believed that such a microbore size can effectively prevent the liquid from entering the mixing channel directly, which may adversely affect the subsequent mixing efficiency and/or lead to unwanted liquid water in the respiratory circuit. However, such a microbore size allows vapour to pass through the nebulizer plate and into the mixing channel.

In an embodiment herein, the micro-humidifier contains a semi-permeable membrane downstream of the mixing channel. The semi-permeable membrane useful herein is available to those skilled in the art and allows water vapour to pass through, while preventing liquid water from passing through. Without intending to be limited by theory, it is believed that such a semi-permeable membrane assists in preventing liquid water from entering the mixing channel.

In an embodiment herein, the liquid feed contains a liquid, preferably water. The water is preferably purified water, but may be other forms of water as well. In an embodiment herein the water is pure water. In another embodiment herein the water contains a drug therein. The drug may be, for example, intended to reduce or prevent inflammation, soothe the patient, increase vascular or bronchial dilation, facilitate sputum excretion, etc. The drug may also be, for example, an antibiotic.

In an embodiment herein, the micro-humidifier is intended to be constantly generating vapour and thus in constant use by the patient. One skilled in the art understands that oftentimes patients may continue to constantly require the inhalation of humidified air for days, week, months, or even years at a time. Some patients may require the use of the micro-humidifier for the rest of their lives. Thus, the small size, weight, and ease of use, reduced chance of contamination, greater control over vapour and gas quality, etc., especially with an automated liquid feed that needs to be changed only a few times a day or less, can greatly improve the quality of treatment for a patient.

It should be understood that the above only illustrates and describes examples whereby the present invention may be carried out, and that modifications and/or alterations may be made thereto without departing from the scope of the invention as defined in the appended claims.

## Claims

1. A micro-humidifier (200) for operably-connecting to a breathing circuit (250), the micro-humidifier (200) comprising:
A. a liquid feed (260);
B. a liquid chamber (216) operably-connected to the liquid feed (260) wherein the liquid chamber (216) has a volume of from about 0.01 mL to about 30 mL;
C. a vapour generator (202) operably-connected to the liquid chamber (216), and
D. a mixing channel (220) downstream of the vapour generator (202), wherein the mixing channel (220) contains a baffle (225) and increased headspace;
wherein the micro-humidifier (200) is designed to be operably-connected to a breathing circuit (250) upstream of the patient, and wherein when the micro-humidifier (200) is operably-connected to a breathing circuit (250) the vapour from the vapour generator (202) enters into the breathing circuit (250).

2. The micro-humidifier (200) according to Claim 1 further comprising a control device (230), wherein the control device (230) comprises a feature selected from the group consisting of a pump system, a temperature sensor, a humidity sensor, an air flow sensor, a timer, an alarm, a circuit board (214), a display screen (232), a control panel, a power source, and a combination thereof.

3. The micro-humidifier (200) according to Claim 1, wherein the micro-humidifier weight is less than or equal to about 300 g.

4. The micro-humidifier (200) according to Claim 1, wherein the vapour generator (202) is selected from the group consisting of a nebulizer, an atomizer, a heating element (211), a sonication apparatus, an electrospray, and a combination thereof.

5. The micro-humidifier (200) according to Claim 1, wherein the liquid feed (260) comprises an infusion set up.

6. The micro-humidifier (200) according to Claim 1, wherein the liquid feed (260) comprises water, and optionally a drug.

7. The micro-humidifier (200) according to Claim 1 wherein the micro-humidifier (200) is detachable from the breathing circuit.

8. The micro-humidifier (200) according to Claim 2 wherein the pump system further comprises a relief valve (512).

9. The micro-humidifier (200) according to Claim 2 wherein the pump system comprises a peristaltic pump (511).

10. The micro-humidifier (200) according to Claim 2, wherein the vapour generator comprises a nebulizer comprising a nebulizer plate (212), and wherein the liquid chamber comprises a heating element (211), wherein the control device comprises a temperature sensor, a humidity sensor, and a circuit board (214), wherein the circuit board is operably-connected to the temperature sensor, the humidity sensor, the nebulizer plate (212) and the heating element (211), wherein the circuit board (214) receives temperature data from the temperature sensor and employs the temperature data to regulate the heating element (211), and wherein the circuit board (214) receives humidity data from the humidity sensor and employs the humidity data to regulate the nebulizer plate (212).

11. The micro-humidifier (200) according to Claim 4 wherein the vapour generator comprises a nebulizer and wherein the micro-humidifier (200) further comprises a heating element (211) and wherein the heating element heats the gas, the liquid, or a combination thereof.

12. A respiratory humidification system comprising:
A. a breathing circuit (250) for operably-connecting a patient and a ventilator (100), and
B. the micro-humidifier (200) according to any one of Claims 1 to 11,
wherein the micro-humidifier (200) is operably-connected to the breathing circuit (250) upstream of the patient, and wherein the vapour from the vapour generator enters into the breathing circuit (250).

## Patentansprüche

1. Mikrobefeuchter (200) zur betriebsmäßigen Verbindung mit einem Atmungskreislauf (250), wobei der Mikrobefeuchter (200) umfasst:
A. eine Flüssigkeitsversorgung (260);
B. eine Flüssigkeitskammer (216), welche betriebsmäßig mit der Flüssigkeitsversorgung (260) verbunden ist, wobei die Flüssigkeitskammer (216) ein Volumen von etwa 0,01 ml bis etwa 30 ml aufweist;
C. einen Dampferzeuger (202), welcher betriebsmäßig mit der Flüssigkeitskammer (216) verbunden ist, und
D. einen Mischkanal (220), welcher stromabwärts des Dampferzeugers (202) angeordnet, ist, wobei
der Mischkanal (220) eine Ablenkplatte (225) und einen vergrößerten Luftraum enthält;
wobei der Mikrobefeuchter (200) ausgebildet ist, um mit einem Atmungskreislauf (250) stromaufwärts des Patienten betriebsmäßig verbunden zu werden, und wobei, wenn der Mikrobefeuchter (200) betriebsmäßig mit einem Atmungskreislauf (250) verbunden ist, der vom Dampferzeuger (202) ankommende Dampf in den Atmungskreislauf (250) eintritt.

2. Mikrobefeuchter (200) nach Anspruch 1, ferner umfassend eine Steuervorrichtung (230), wobei die Steuervorrichtung (230) ein Merkmal umfasst, ausgewählt aus der Gruppe bestehend aus einem Pumpsystem, einem Temperatursensor, einem Feuchtigkeitssensor, einem Luftstromsensor, einem Zeitgeber, einem Alarm, einer Leiterplatte (214), einem Anzeigebildschirm (232), einem Steuerpult, einer Energiequelle, und einer Kombination davon.

3. Mikrobefeuchter (200) nach Anspruch 1, wobei das Gewicht des Mikrobefeuchters etwa 300 g oder weniger beträgt.

4. Mikrobefeuchter (200) nach Anspruch 1, wobei der Dampferzeuger (202) aus der Gruppe ausgewählt ist, bestehend aus einem Vernebler, einem Zerstäuber, einem Heizelement (211), einer Beschallungsvorrichtung, einem Elektrospray, und einer Kombination davon.

5. Mikrobefeuchter (200) nach Anspruch 1, wobei die Flüssigkeitsversorgung (260) eine Infusionseinrichtung umfasst.

6. Mikrobefeuchter (200) nach Anspruch 1, wobei die Flüssigkeitsversorgung (260) Wasser und optional ein Arzneimittel umfasst.

7. Mikrobefeuchter (200) nach Anspruch 1, wobei der Mikrobefeuchter (200) vom Atmungskreislauf lösbar ist.

8. Mikrobefeuchter (200) nach Anspruch 2, wobei das Pumpsystem ferner ein Überdruckventil (512) umfasst.

9. Mikrobefeuchter (200) nach Anspruch 2, wobei das Pumpsystem eine Schlauchpumpe (511) umfasst.

10. Mikrobefeuchter (200) nach Anspruch 2, wobei der Dampferzeuger einen Vernebler umfasst, der eine Verneblerplatte (212) umfasst, und wobei die Flüssigkeitskammer ein Heizelement (211) umfasst, wobei die Steuervorrichtung einen Temperatursensor, einen Feuchtigkeitssensor und eine Leiterplatte (214) umfasst, wobei die Leiterplatte mit dem Temperatursensor, dem Feuchtigkeitssensor, der Verneblerplatte (212) und dem Heizelement (211) betriebsmäßig verbunden ist, wobei die Leiterplatte (214) Temperaturdaten vom Temperatursensor empfängt und die Temperaturdaten verwendet, um das Heizelement (211) zu regeln, und wobei die Leiterplatte (214) Feuchtigkeitsdaten vom Feuchtigkeitssensor empfängt und die Feuchtigkeitsdaten verwendet, um die Verneblerplatte (212) zu regeln.

11. Mikrobefeuchter (200) nach Anspruch 4, wobei der Dampferzeuger einen Vernebler umfasst und wobei der Mikrobefeuchter (200) ferner ein Heizelement (211) umfasst und wobei das Heizelement ein Gas, eine Flüssigkeit, oder eine Kombination davon aufwärmt.

12. Atmungsbefeuchtungssystem, umfassend:
A. einen Atmungskreislauf (250) zur betriebsmäßigen Verbindung mit einem Patienten und einem Ventilator (100), und
B. den Mikrobefeuchter (200) nach einem der Ansprüche 1 bis 11,
wobei der Mikrobefeuchter (200) betriebsmäßig mit dem Atmungskreislauf (250) stromaufwärts des Patienten verbunden ist, und wobei der Dampf aus dem Dampferzeuger in den Atmungskreislauf (250) eintritt.

## Revendications

1. Micro-humidificateur (200) pour une connexion de façon opérationnelle sur un circuit de respiration (250), le micro-humidificateur (200) comprenant :
A. une alimentation en liquide (260) ;
B. une chambre de liquide (216) qui est connectée de façon opérationnelle à l'alimentation en liquide (260), dans lequel la chambre de liquide (216) présente un volume qui va d'environ 0,01 mL à environ 30 mL ;
C. un générateur de vapeur (202) qui est connecté de façon opérationnelle à la chambre de liquide (216) ; et
D. un canal de mélange (220) en aval du générateur de vapeur (202), dans lequel le canal de mélange (220) contient une chicane (225) et un espace de tête augmenté ;
dans lequel :
le micro-humidificateur (200) est conçu de manière à ce qu'il soit connecté de façon opérationnelle à un circuit de respiration (250) en amont du patient, et dans lequel, lorsque le micro-humidificateur (200) est connecté de façon opérationnelle à un circuit de respiration (250), la vapeur en provenance du générateur de vapeur (202) entre à l'intérieur du circuit de respiration (250).

2. Micro-humidificateur (200) selon la revendication 1, comprenant en outre un dispositif de commande (230), dans lequel le dispositif de commande (230) comprend une caractéristique qui est sélectionnée au sein du groupe qui est constitué par un système de pompe(s), un capteur de température, un capteur d'humidité et un capteur d'écoulement d'air, un temporisateur, une alarme, une carte de circuit (214), un écran d'affichage (232), un panneau de commande, une source d'alimentation et une combinaison de ceux-ci.

3. Micro-humidificateur (200) selon la revendication 1, dans lequel le poids du micro-humidificateur est inférieur ou égal à environ 300 g.

4. Micro-humidificateur (200) selon la revendication 1, dans lequel le générateur de vapeur (202) est sélectionné au sein du groupe qui est constitué par un nébuliseur, un atomiseur, un élément de chauffage (211), un appareil de sonication, un dispositif d'électropulvérisation et une combinaison de ceux-ci.

5. Micro-humidificateur (200) selon la revendication 1, dans lequel l'alimentation en liquide (260) comprend une installation de perfusion.

6. Micro-humidificateur (200) selon la revendication 1, dans lequel l'alimentation en liquide (260) comprend de l'eau et en option, un médicament.

7. Micro-humidificateur (200) selon la revendication 1, dans lequel le micro-humidificateur (200) peut être détaché du circuit de respiration.

8. Micro-humidificateur (200) selon la revendication 2, dans lequel le système de pompe(s) comprend en outre une soupape de surpression (512).

9. Micro-humidificateur (200) selon la revendication 2, dans lequel le système de pompe(s) comprend une pompe péristaltique (511).

10. Micro-humidificateur (200) selon la revendication 2, dans lequel le générateur de vapeur comprend un nébuliseur qui comprend une plaque de nébuliseur (212), et dans lequel la chambre de liquide comprend un élément de chauffage (211), dans lequel le dispositif de commande comprend un capteur de température, un capteur d'humidité et une carte de circuit (214), dans lequel la carte de circuit est connectée de façon opérationnelle au capteur de température, au capteur d'humidité, à la plaque de nébuliseur (212) et à l'élément de chauffage (211), dans lequel la carte de circuit (214) reçoit des données de température en provenance du capteur de température et utilise les données de température pour régler l'élément de chauffage (211), et dans lequel la carte de circuit (214) reçoit des données d'humidité en provenance du capteur d'humidité et utilise les données d'humidité pour régler la plaque de nébuliseur (212).

11. Micro-humidificateur (200) selon la revendication 4, dans lequel le générateur de vapeur comprend un nébuliseur et dans lequel le micro-humidificateur (200) comprend en outre un élément de chauffage (211) et dans lequel l'élément de chauffage chauffe le gaz, le liquide ou une combinaison de ceux-ci.

12. Système d'humidification de respiration comprenant :
A. un circuit de respiration (250) destiné à être connecté de façon opérationnelle sur un patient et sur un ventilateur (100) ; et
B. le micro-humidificateur (200) selon l'une quelconque des revendications 1 à 11, dans lequel le micro-humidificateur (200) est connecté de façon opérationnelle au circuit de respiration (250) en amont du patient, et dans lequel la vapeur en provenance du générateur de vapeur entre à l'intérieur du circuit de respiration (250).
